# EUROPÄISCHE PATENTSCHRIFT

(11) **EP 2 773 936 B1**
(45) Veröffentlichungstag und Bekanntmachung des Hinweises auf die Patenterteilung: **12.05.2021**
(21) Anmeldenummer: 12806650.3
(22) Anmeldetag: 05.11.2012
(51) Int. Cl.: G01M 3/32, G01M 3/34, G01M 3/36

(54) **VERFAHREN UND VORRICHTUNG ZUR PRÜFUNG VON KONTINUIERLICH ZUGEFÜHRTEN GEGENSTÄNDEN**
METHODS AND DEVICE FOR INSPECTING CONTINUOUSLY FED OBJECTS
PROCÉDÉ ET DISPOSITIF DE CONTRÔLE D'OBJETS ACHEMINÉS EN CONTINU

(30) Priorität: 04.11.2011 DE 202011107446 U
(43) Veröffentlichungstag der Anmeldung: 10.09.2014
(73) Patentinhaber: Polman, Eckhard, 46569 Hünxe (DE)
(72) Erfinder: Polman, Eckhard, 46569 Hünxe (DE)
(74) Vertreter: Grosse Schumacher Knauer von Hirschhausen
(86) Internationale Anmeldenummer: PCT/IB2012/002235
(87) Internationale Veröffentlichungsnummer: WO 2013/064889

(56) Entgegenhaltungen:
- EP-A2- 2 187 192
- WO-A2-2004/056655
- DE-A1- 1 573 478
- NL-A- 8 900 299
- US-A- 700 891
- US-A- 3 824 840

## Beschreibung

Die Erfindung betrifft ein Verfahren gemäß Anspruch 1 und eine Vorrichtung gemäß Anspruch 12 zur vakuumbasierten Dichtheitsprüfung von in einem kontinuierlichen Strom zugeführten Gegenständen.

Die Prüfung von Gegenständen wie etwa Verpackungen und Bauteilen auf Qualitätskriterien wie etwa Dichtheit oder Maßhaltigkeit ist auf vielen technischen Gebieten erforderlich. Die bekannten Verfahren und Vorrichtungen sind jedoch häufig komplex, langsam, wartungsanfällig und diskontinuierlich sowie hinsichtlich der Prüfmöglichkeiten beschränkt. Dies soll zunächst anhand der Prüfung von Verpackungen auf Dichtheit veranschaulicht werden.

Die Prüfung von Verpackungen auf Dichtheit ist auf vielen technischen Gebieten erforderlich. Beispielsweise muss bei Lebensmittelverpackungen sichergestellt sein, dass der Inhalt und eine ggf. vorhandene Schutzatmosphäre nicht austreten können sowie das Innere der Verpackung nicht durch Eindringen von Fremdkörpern oder Gasen kontaminiert wird. Andernfalls wird das Lebensmittel ungenießbar oder es verkürzt sich die Lebensdauer.

Verpackungen für andere Waren als Lebensmittel müssen ebenfalls dicht sein, um einen Warenschwund durch Auslaufen und eine Verschmutzung zu vermeiden. Typische Verpackungen, die auf Dichtheit geprüft werden müssen, sind Getränkeverpackungen aller Art, Frischfleischverpackungen aller Art, Blisterverpackungen, auch für Medikamente, eingepackte oder verschweißte Lebensmittelverpackungen, Tetrapacks, Kunststoffbecher wie etwa Joghurtbecher mit aufgeschweißtem oder aufgeklebtem Deckel, Getränkeflaschen, Ölbehälter, Farbbehälter, Tuben, Flaschen und dergleichen mehr.

Bei der Massenherstellung derartiger verpackter Waren ist die Aufgabe zu lösen, jede Verpackung und nicht lediglich Stichproben auf Dichtheit zu prüfen.

Hierzu sind getaktete Verfahren sowie Vorrichtungen zur Durchführung dieser Verfahren bekannt, bei denen jeweils eine Anzahl von Verpackungen zusammen in einer Prüfkammer auf Dichtheit geprüft wird. Damit ist jedoch eine Reihe von Nachteilen verbunden. So müssen die Verpackungen auf einer Palette angeordnet und dann in die Prüfkammer verbracht werden, dort die Prüfung durchlaufen, aus der Prüfkammer entnommen werden und anschließend einer weiteren Bearbeitung, beispielsweise der Etikettierung oder der Anordnung in Verpackungseinheiten, zugeführt werden. Üblicherweise werden bei der Massenherstellung derartige Verpackungen in einem kontinuierlichen Strom, z.B. auf einem Band oder dergleichen, befördert, wobei das Band zum Befüllen und Verschließen kurz gestoppt wird. Dieser kontinuierliche Verpackungsstrom muss unterbrochen werden, um eine Anzahl von Verpackungen auf einer Palette für die Dichtheitsprüfung zu sammeln, und anschließend müssen die Verpackungen zur weiteren Verarbeitung wieder von der Palette in einen Verpackungsstrom überführt werden. Eine Zeitverzögerung und ein erheblicher technischer Aufwand sind die Folge. Zudem lassen sich als undicht erkannte Verpackungen nur mit hohem technischen Aufwand aus der Palette entfernen.

Die bekannten Vorrichtungen und Verpackungen sind aufgrund der technischen Komplexität teuer sowie wartungsanfällig und beim Übergang vom kontinuierlichen Verpackungsstrom zur getakteten Prüfung und danach wieder in den kontinuierlichen Verpackungsstrom steigt das Risiko für Ausfälle, da die Verpackungen mechanisch mehrfach umgeordnet werden müssen.

Dieselben Nachteile ergeben sich bei der Prüfung von Gegenständen im Allgemeinen, wie etwa Spritzgussteilen oder auch komplexen mechanischen oder elektrischen Bauteilen, z.B. Computerkomponenten, Roboterkomponenten, Sensoren, Aktoren, Bildgebern, Fotoapparaten, Besteckteilen usw., die einem mechanischen, elektrischen, thermischen, magnetischen, geruchsbasierten, vakuumbasierten und/oder druckbasierten und/oder anderweitigen Stimulus wie etwa Ein- und Ausschalten, Aufnahme eines Fotos, Messung einer Temperatur etc., ausgesetzt werden müssen, um ein Qualitätskriterium zu bestimmen und fehlerhafte Gegenstände von fehlerfreien zu trennen.

NL 8 900 299 A beschreibt ein automatisches System zur Detektion von Leckagen in gasdichten Verpackungen mittels Druckmessungen.

DE 15 73 478 A1 beschreibt ein Verfahren und eine Prüfeinrichtung zur serienweisen, automatischen Prüfung von Hohlkörpern auf Dichtheit mittels Vakuumbeaufschlagung und Erfassung der Druckdifferenz.

US 700 891 A beschreibt eine Dosen-Testvorrichtung, bei der die zu testenden, unter Vakuum angesaugten Dosen an einer kreisförmigen Halterung durch ein Flüssigkeitsbad gezogen werden und bei vorliegendem Leck aufgrund des Unterdruckverlustes aus der Halterung fallen.

US 3 824 840 A beschreibt eine Vorrichtung zum Aussortieren von zerbrechlichen Bechern mittels einer Drucküberwachung eines abdichtenden Kontaktes zwischen einer Membran und der zu untersuchenden Bechern.

EP 2 187 192 A2 beschreibt ein Leckdetektionssystem in Lebensmittelverpackungen und ein entsprechendes Verfahren.

WO 2004/056655 A2 beschreibt eine Vakuumverpackungsvorrichtung und ein entsprechendes Verfahren, bei der die Verpackungen entlang eines Transportbandes mittels einem Paar Hitzestäbe und Hitzeambosspaar versiegelt und durch eines gezackten Messers voneinander getrennt werden.

Ausgehend hiervon liegt der Erfindung die Aufgabe zugrunde, ein Verfahren gemäß Anspruch 1 und eine Vorrichtung gemäß Anspruch 12 zur vakuumbasierten Dichtheitsprüfung von in einem kontinuierlichen Strom zugeführten Gegenständen zu schaffen, die eine 100%-Kontrolle ohne Zeitverlust schnell, kostengünstig und in technischer Hinsicht ausfallsicher und wartungsarm, also zuver-Diese Aufgabe wird entsprechend den Merkmalen der Ansprüche 1 und 12 gelöst.

Demnach wird ein Verfahren gemäß Anspruch 1 zur vakuumbasierten Dichtheitsprüfung von in einem kontinuierlichen Strom zugeführten Gegenständen, insbesondere Verpackungen oder Bauteilen, geschaffen, bei dem entlang einer Prüfstrecke ein Prüfkörper mit einem Gegenstand mitgeführt wird und mittels eines Sensors eine Reaktion auf eine Prüfung des Gegenstands erfasst wird, um festzustellen, ob der Gegenstand eine Qualitätskriterium erfüllt, insbesondere dicht oder undicht oder maßhaltig oder funktionsfähig ist. Damit ist eine schnelle kontinuierliche Prüfung von Gegenständen realisierbar.

Vorzugsweise veranlasst Prüfkörper eine Prüfung des Gegenstands. Beispielsweise kann der Prüfkörper den Gegenstand mit einem Vakuum, einem Druck und/oder einem mechanischen, akustischen, optischen, thermischen und/oder elektrischen oder sonstigen Testimpuls beaufschlagen und/oder den Gegenstand oder einen Teil davon betätigen, auslenken, befüllen, entleeren, ein- und/oder ausschalten, oder anderweitig stimulieren.

Zweckmäßigerweise kann der Prüfkörper den Sensor umfassen, um unmittelbar die Reaktion auf eine Stimulation zu erfassen. Beispielsweise kann bei mechanischer Stimulation, z.B. Verformen oder Betätigen des Gegenstands durch einen mechanischen Testgegenstand, der Druck zum Verformen oder Betätigen direkt gemessen werden, was z.B. einen Hinweis auf einen undichten Gegenstand geben kann, der infolge Austritts eingeschlossenen Gases einfacher verformbar ist als ein dichter Gegenstand, bei dem das eingeschlossene Gas nicht austreten kann. Der Sensor kann aber auch außerhalb des Prüfkörpers angeordnet sein. Selbstverständlich können mehrere, insbesondere verschiedene, Sensoren verwendet werden.

Mittels des Sensors kann eine optische, akustische, thermische, mechanische oder sonstige Reaktion des Gegenstands erfasst und auf ein Qualitätskriterium hin geprüft werden.

In einer Ausführungsform wird ein Verfahren zur vakuumbasierten Dichtheitsprüfung von in einem kontinuierlichen Verpackungsstrom zugeführten Verpackungen, insbesondere Lebensmittelverpackungen, geschaffen, bei dem entlang einer Prüfstrecke ein Dichtkörper mit einer Verpackung mitgeführt und mit Vakuum beaufschlagt wird und mittels eines Sensors eine Reaktion auf die Beaufschlagung der Verpackung mit Vakuum erfasst wird, um festzustellen, ob die Verpackung dicht oder undicht ist. Hierdurch kann die Dichtheitsprüfung in den Fertigungsprozess an einer beliebigen Stelle integriert werden, ohne den Fertigungsprozess zu beeinflussen. Beispielsweise kann direkt nach der Abfüllung und dem Verschließen die Dichtheitsprüfung erfolgen, während die Verpackungen einer weiteren Verarbeitungsstation wie etwa der Etikettierung in einem kontinuierlichen Verpackungsstrom zugeführt werden. Es ergibt sich eine Verringerung der technischen Komplexität und es müssen keine Verpackungen aus dem Verpackungsstrom entnommen und nach der Dichtheitsprüfung wieder in diesen eingefügt werden. Eine 100%-Kontrolle, also eine Kontrolle jeder einzelnen Verpackung, ist ohne Zeitverlust schnell, kostengünstig und in technischer Hinsicht ausfallsicher und wartungsarm möglich.

Der kontinuierliche Verpackungsstrom ist dabei beispielsweise durch ein Fließband oder einen Endlosförderer realisiert. Die Verpackungen werden dabei aufeinanderfolgend in einer Reihe oder ggf. mehrere nebeneinander in einer Transportrichtung bewegt. Die Bewegung ist kontinuierlich, d.h. fließend, wobei der Begriff "kontinuierlich" für diese Anmeldung auch die Änderung der Geschwindigkeit bis zum Stillstand, also auch einen quasi-kontinuierlichen Transport umfasst.

Während des Transports durchlaufen die Verpackungen eine Prüfstrecke. Dort wird ein Dichtkörper mit den Verpackungen mitgeführt. Zweckmäßigerweise ist ein Dichtkörper für eine Verpackung vorgesehen, jedoch können zwei oder mehr Verpackungen gleichzeitig mit einem Dichtkörper geprüft werden, der dann entsprechend groß auszugestalten ist. Zweckmäßig ist die Verwendung einer Vielzahl von Dichtkörpern, die mit den Verpackungen entlang der Prüfstrecke mitgeführt werden. So können mehrere Verpackungen gleichzeitig einer Dichtheitsprüfung unterzogen werden.

Mit den Dichtkörpern wird ein Vakuum zur Prüfung der Verpackungen erzeugt. Als Reaktion auf die Beaufschlagung mit Vakuum wird eine Verformung der Verpackung, ein Druckanstieg im Dichtkörper, eine verlangsamte Vakuumbildung im Dichtkörper, ein Gas im Dichtkörper und/oder dergleichen erfasst.

Wird die Verformung erfasst, erfolgt dies unter der Annahme, dass sich eine undichte Verpackung nicht so stark aufbläht wie eine dichte Verpackung oder gänzlich unverformt bleibt. Dies kann mit einem optischen Sensor, z.B. einer Digitalkamera mit Auswertesoftware, erfasst werden. Derartige optische Sensoren sind in der Warenprüfung bekannt. Es kann auch ein Überschreiten einer Verformungsgrenze mit einem derartigen optischen Sensor oder einfach mit einer Lichtschranke erfasst werden, die bei ausreichender Verformung unterbrochen wird und so eine dichte Verpackung signalisiert. Beliebige optische Sensoren können hierfür verwendet werden.

Wird der Druckanstieg im Dichtkörper oder eine verlangsamte Vakuumbildung erfasst, erfolgt dies unter der Annahme, dass Gas aus der undichten Verpackung entweicht und der Vakuumbildung entgegenwirkt. Ist die Vakuumbildung verlangsamt oder verzögert, deutet dies auf eine Undichtigkeit hin, die bereits bestanden hat; beispielsweise ist die Verpackung fehlerhaft verschlossen worden oder hatte mechanische Defekte. Steigt der Druck plötzlich an, deutet dies auf eine im Unterdruck entstandene Undichtigkeit hin; beispielsweise hält eine Verschweißung oder ein anderer Verschluss oder die Verpackung nicht den erforderlichen mechanischen Belastungen stand. Damit ist auch eine Diagnose der Ursache der Undichtigkeit möglich. Hierzu können Drucksensoren verwendet werden, mit denen der Ist-Druck gemessen werden kann. Es kann auch ein Durchflusssensor verwendet werden, mit dem die Menge an abgesaugter Luft erfasst werden kann; fließt nach einer bestimmten Zeitspanne noch immer Gas durch den Durchflusssensor, deutet dies auf eine undichte Verpackung hin. Auch kann ein Druckschalter verwendet werden, um bei Erreichen eines bestimmten Vakuums die Dichtheit zu signalisieren. Mehrere gleiche oder unterschiedliche Sensoren können kombiniert werden.

Schließlich kann ein Gasdetektor verwendet werden, um Gas im Dichtkörper zu erkennen, welches eigentlich z.B. als Schutzgas in der Verpackung verbleiben sollte und nur bei einer undichten Verpackung aus dieser entweichen kann. Da das in der Verpackung enthaltene Gas bekannt ist, kann ein gezielt darauf abgestimmter kostengünstiger Gasdetektor verwendet werden.

Um ein Vakuum erzeugen zu können, wird die Verpackung angehoben und/oder der Dichtkörper wird abgesenkt. Hierzu können entsprechende Mechanismen vorgesehen sein. Vorzugsweise sind die Verpackungen auf einer Unterglocke angeordnet, und der Dichtkörper dichtet mit der Unterglocke ab, wobei die Verpackung, oder ggf. mehrere Verpackungen gleichzeitig, zwischen Dichtkörper und Unterglocke eingeschlossen ist bzw. sind. Die Unterglocke kann dabei ein Gummiteller beliebiger, insbesondere runder oder eckiger Form sein, auf dem die Verpackung sich befindet. Auch kann die Unterglocke ein Ring beliebiger, insbesondere runder oder eckiger Form sein, in dessen inneren sich die Verpackung befindet. Ggf. ist die Fördervorrichtung, auf der die Verpackungen transportiert werden, aus einem Material, gegenüber welchem die Dichtkörper abdichten können, so dass die Fördervorrichtung die Unterglocke bildet.

Je nach Form und Verschluss der Verpackung kann es auch zweckmäßig sein, dass die Oberglocke gegenüber der Verpackung und nicht gegenüber einer Unterglocke abdichtet. Beispielsweise kann die Oberglocke mit einem Kanisterhals in dichtenden Eingriff dergestalt gebracht werden, dass der Kanisterverschluss unter Vakuumeinfluss gebracht werden kann. Bei einer flächigen Verpackung mit Sichtfenster kann die Oberglocke das Sichtfenster umgebend mit der Verpackung in dichtenden Eingriff gebracht werden. Wichtig ist, dass ein Teil des Verschlusses oder vorzugsweise der gesamte Verschluss einem Vakuum ausgesetzt werden können, wobei die Oberglocke gegenüber der Verpackung und/oder einer Unterglocke, die Teil der Fördervorrichtung sein kann, abdichtet.

Eine perfekte Abdichtung ist zwar vorteilhaft, aber nicht zwingend. So kann eine Undichtigkeit der Abdichtung durchaus toleriert werden, solange sie unterhalb eines Grenzwerts liegt, der eine Verformung einer undichten Verpackung, einen Gas- oder Materialaustritt daraus oder eine feststellbare Verlangsamung der Vakuumerzeugung sicherstellt.

Besonders zweckmäßig ist es, wenn der Verpackungsstrom auf einem Endlosförderer zugeführt wird und/oder der Dichtkörper an einem Endlosförderer umläuft. Derartige Endlosförderer sind im Stand der Technik bekannt. Sie sind robust und zuverlässig.

Die Verwendung von eines Endlosförderers als Transportmittel für die Verpackungen und/oder eines Endlosförderers, an dem der oder die Dichtkörper angeordnet sind, ist besonders vorteilhaft, wenn der Endlosförderer einen integrierten Energiekanal aufweist, der mit pneumatischer Energie beaufschlagbar ist, die als Vakuum zur Gasentleerung des Dichtkörpers entnommen werden kann. Derartige Endlosförderer sind beispielsweise aus der WO 2008/104404 A2 und in Form einer besonders langlebigen und wartungsarmen Blockkette aus der WO 2010/089101 A1 bekannt. Bei derartigen Endlosförderern wird einem endlos umlaufenden Zahnriemen oder einer endlos umlaufenden Blockkette beispielsweise an Umlenkrollen pneumatische Energie, insbesondere Druckluft, aber ggf. auch Vakuum, zugeführt. Die pneumatische Energie ist in einem Kanal, der sich entlang des Endlosförderers insbesondere darin erstreckt, verfügbar und an jeder Stelle abgreifbar. So können die Dichtkörper die pneumatische Energie aus dem Kanal insbesondere gesteuert entnehmen, um ein Vakuum zu erzeugen, wenn ein Dichtkörper mit einer Verpackung mitgeführt wird. Das Vakuum wird vorzugsweise aus der Druckluft im Kanal über Venturidüsen erzeugt. Derartige Endlosförderer wiesen vorzugsweise auch einen Energiestrang für elektrische Energie auf, so dass elektrisch betätigte Elemente und Sensoren mit elektrischer Energie versorgt werden können und zudem elektrische Signale wie etwa Messwerte und Steuersignale zwischen einer Steuerung und/oder Auswerteeinheit und den Sensoren übertragen werden können. Die Dichtkörper können auch mit einer Einheit oder mehreren Einheiten am Endlosförderer verbunden sein, die eine Ansteuerung und/oder Auswertung der Sensoren sowie ggf. eine Ansteuerung der Vakuumerzeugung veranlassen; dies kann allerdings auch außerhalb des Endlosförderers in einer zentralen Steuerung erfolgen.

Besonders vorteilhaft ist es, wenn undichte Verpackungen auf dem Endlosförderer gehalten und insbesondere im Rücklauf abgeworfen werden. Damit ist eine Ausschleusung undichter Verpackungen aus dem Verpackungsstrom möglich.

Dies kann durch elektrisch und/oder pneumatisch betätigte Greifer, Saugglocken oder dergleichen am die Dichtkörper aufweisenden Endlosförderer erfolgen, indem die undichten Verpackungen aufgenommen und damit dem Verpackungsstrom entnommen und an geeigneter Stelle, zweckmäßigerweise nach dem Rücklauf, abgeworfen werden. Undichte Verpackungen können statt dessen auch seitlich herausgeschoben werden mittels entsprechender Auswurfvorrichtungen, die anstelle der Greifer am Endlosförderer oder stationär an einer entsprechenden Stelle nach der Prüfstrecke vorgesehen sein können.

Auch können die Verpackungen auf dem Endlosförderer, auf welchem der Verpackungsstrom verläuft, mittels Vakuum oder Greifern oder dergleichen gehalten werden, bis sie um die Umlenkrolle herum im Rücklauf sich befinden, und dort abgeworfen werden, während die dichten Verpackungen an einer anderen Stelle abgeworfen werden, oder umgekehrt.

Die Erfindung schafft ferner eine Vorrichtung zur vakuumbasierten kontinuierlichen Dichtheitsprüfung von in einem kontinuierlichen Verpackungsstrom zugeführten Verpackungen, insbesondere Lebensmittelverpackungen, bei der ein entlang einer Prüfstrecke mit einer Verpackung mitführ- und mit Vakuum beaufschlagbarer Dichtkörper vorgesehen ist und ein Sensor zum Erfassen einer Reaktion auf die Beaufschlagung der Verpackung mit Vakuum vorgesehen und mit einer Auswerteeinheit gekoppelt ist, die anhand der erfassten Reaktion die Verpackung als dicht oder undicht erkennen kann. Der Sensor kann als Reaktion auf die Beaufschlagung mit Vakuum eine Verformung der Verpackung, ein Druckanstieg im Dichtkörper, eine verlagsamte Vakuumbildung im Dichtkörper, ein Gas im Dichtkörper und/oder dergleichen erfassen.

Ein Mechanismus zum Anheben der Verpackung und/oder ein Mechanismus zum Absenken der Dichtkörper kann vorgesehen sein.

Die Verpackungen können auf einer Unterglocke angeordnet sein, wobei der Dichtkörper mit der Unterglocke abdichtet und die Verpackung zwischen Dichtkörper und Unterglocke eingeschlossen ist.

Ein Endlosförderer zum Zuführen des Verpackungsstrom kann vorgesehen sein und/oder der Dichtkörper kann an einem Endlosförderer befestigt sein und mit diesem umlaufen.

Der Endlosförderer kann einen integrierten Energiekanal aufweisen, der mit pneumatischer Energie beaufschlagbar ist, die als Vakuum zur Gasentleerung des Dichtkörpers entnehmbar ist.

Der Endlosförderer kann einen Haltemechanismus für die Verpackungen aufweisen und eine Steuerung kann vorgesehen sein, mit der undichte Verpackungen auf dem Endlosförderer gehalten und im Rücklauf abgeworfen werden.

Ein optischer Sensor, ein Drucksensor, ein Gassensor, ein Durchflusssensor, ein Druckschalter und/oder dergleichen können zum Erfassen einer Reaktion auf die Beaufschlagung der Verpackung mit Vakuum vorgesehen sein.

Die Erfindung schafft ferner eine Verwendung eines Endlosförderers mit Energieversorgungsstrang für pneumatische Energie, wie er beispielsweise aus der WO 2010/089101 A1 bekannt ist, zur Durchführung des erfindungsgemäßen Verfahrens bzw. für die erfindungsgemäße Vorrichtung.

Weitere Ausgestaltungen sind insbesondere den unabhängigen Ansprüchen zu entnehmen und sind jeweils eigenständig erfinderisch.

Weitere Ausgestaltungen, Eigenschaften und Details der Erfindung sind der nachfolgenden Beschreibung und Figuren sowie der Ansprüche zu entnehmen.
Fig. 1 illustriert eine Vorrichtung gemäß Anspruch 12 zur vakuumbasierten Dichtheitsprüfung von in einem kontinuierlichen Strom bzw. Verpackungsstrom zugeführten Gegenständen, insbesondere Verpackungen.
Fig. 2 illustriert eine weitere Ausführungsform einer erfindungsgemäßen Vorrichtung.
Fig. 3 illustriert eine weitere Ausführungsform einer erfindungsgemäßen Vorrichtung.
Fig. 4 illustriert eine Ausführungsform mit einem Rundtisch.
Fig. 5 und 6 illustrieren eine schaufelradähnliche Ausführungsform.
Fig. 7 illustriert eine weitere Ausführungsform.

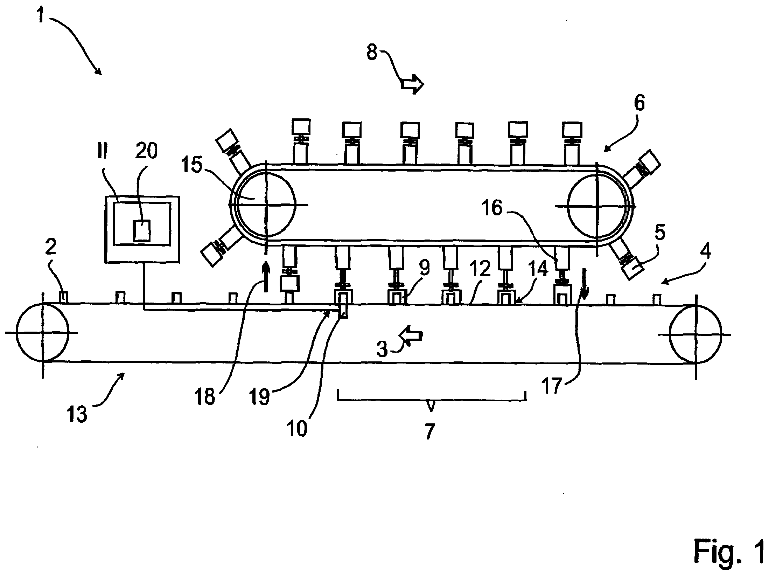

Die in Fig. 1 dargestellte Vorrichtung 1 gemäß Anspruch 12 zur vakuumbasierten Dichtheitsprüfung von in einem kontinuierlichen Strom bzw. Verpackungsstrom zugeführten Gegenständen, insbesondere Verpackungen 2, die in einem entlang eines Pfeils 3 verlaufenden Verpackungsstrom 4 kontinuierlich bewegt werden, umfasst Dichtkörper 5, die beispielhaft auf einem Endlosförderer 6 entlang einer Prüfstrecke 7 synchron mit dem Verpackungsstrom 4 in Richtung eines Pfeils 8 mitlaufen und die Verpackungen mit einem Vakuum 9 beaufschlagen, wobei ein Sensor 10 zum Erfassen einer Reaktion auf die Beaufschlagung einer Verpackung 2 mit Vakuum vorgesehen und mit einer Auswerteeinheit 11 gekoppelt ist, die anhand der erfassten Reaktion die Verpackung 2 als dicht oder undicht erkennen kann.

Der Verpackungsstrom 4 liefert Verpackungen hinter- und ggf. auch nebeneinander, hier auf einer Förderfläche 12 eines Endlosförderers 13, die im vorherigen Verarbeitungsschritt mit Ware wie etwa Lebensmittel befüllt und luftdicht verschlossen, z.B. mit durchsichtigem Material verschweißt, worden sind. Der Verpackungsstrom 4 ist kontinuierlich, d.h. er bewegt sich in Richtung des Pfeils 3 mit vorzugsweise konstanter Geschwindigkeit, die sich jedoch ggf. ändern kann und wobei auch Stillstandszeiten, insbesondere getaktet, vorgesehen sein können. Wichtig ist, dass die Dichtkörper 5, die beispielsweise als insbesondere durchsichtige Saugglocken ausgestaltet sein können, zumindest im Bereich der Prüfstrecke 7 synchron zu den Verpackungen 2 bewegt werden, also erforderlichenfalls ebenfalls mit veränderbarer Geschwindigkeit oder mit Stillstandszeiten.

Während des Gleichlaufs entlang der Prüfstrecke 7 senken sich im dargestellten Beispiel die Dichtkörper 5 ab, um mit einer Unterglocke 14, die separat ausgestaltet oder wie dargestellt die Oberfläche 12 sein kann, abzudichten.

Vorzugsweise wird dann das Vakuum 9 erzeugt; ggf. erzeugen die Dichtkörper 5 aber bereits kurz vorher oder ununterbrochen einen Unterdruck. Die Energie hierfür kann aus dem Endlosförderer 6 stammen, der vorzugsweise mit einem Kanal ausgestaltet ist, welcher sich endlos oder entlang von Abschnitten im Endlosförderer 6 entlang seiner Längsrichtung erstreckt und beispielsweise mit Druckluft beaufschlagt sein kann. Die Druckluft wird vorzugsweise an einer Umlenkung 15 axial eingespeist und radial dem Kanal im Bereich der Auflage zwischen Umlenkung 15 und Endlosförderer 6 zugeführt, wie dies für derartige Endlosförderer 6 bekannt ist. Aus der Druckluft wird z.B. über Venturidüsen das Vakuum 9 erzeugt. Die Druckluft kann auch den Absenk- und Hebemechanismus 16 zum Absenken entlang des Pfeils 17 zu Beginn der Prüfstrecke 7 bzw. Anheben entlang des Pfeils 18 am Ende der Prüfstrecke 7 bewirken, was zweckmäßigerweise ventilgesteuert erfolgt.

Die dargestellte Vorrichtung 1 umfasst hier genau eine hier ortsfeste Messstelle 19, an der der Sensor 10 und ggf. weitere Sensoren die Reaktion auf die Beaufschlagung mit Vakuum erfasst bzw. erfassen. Die Messstelle muss jedoch nicht ortsfest sein und es können mehrere Messstellen vorgesehen sein. Beispielhaft ist ein optischer Sensor 10 illustriert, mit dem eine Verformung der Verpackung 2 an der Messstelle 19 erfasst werden kann, was durch die Abbildung 20 in der Auswerteeinheit 11 illustriert ist. Ggf. kann ein akustischer Sensor die Geräuschentwicklung erfassen, oder ein Drucksensor den Druckabfall im Dichtkörper durch Austreten von Luft oder Gas aus einer undichten Verpackung etc. Die Auswerteeinheit 11 ist zweckmäßigerweise eine entsprechend ausgestaltete Computereinheit. Der Sensor 10 kann auch ein Drucksensor usw. sein und es können an der Messstelle 19 mehrere insbesondere verschiedene Sensoren vorgesehen sein. Anstelle des ortsfesten Sensors 10 oder zusätzlich kann an oder in den Dichtkörpern 5 ein Sensor oder mehrere Sensoren vorgesehen sein. Diese Sensoren können mit der Auswerteeinheit 11 über einen elektrischen Energiestrang im Endlosförderer 6 und Abgriffen an der Umlenkrolle 15 und/oder per Funk gekoppelt sein. Die Auswerteeinheit 11 erkennt nunmehr anhand der Sensordaten, ob eine Verpackung 2 dicht oder undicht ist. Eine Ausschleusung undichter Verpackungen 2 aus dem Verpackungsstrom 4 kann veranlasst werden. - In anderen Anwendungen kann der Sensor beispielsweise erfassen, ob sich ein zu prüfendes Bauteil wie gewünscht bewegt, oder ob eine zu prüfende LED-Baugruppe Licht abgibt, oder ob ein Brandschutzmelder auf in den Dichtkörper einzulassenden Rauch akustisch und/oder optisch reagiert oder dergleichen; der Sensor und/oder der Aktor für die Prüfung auf ein derartiges Qualitätskriterium können je nach durchzuführender Prüfung am, innerhalb und/oder außerhalb des Prüfkörpers, der kein Dichtkörper sein muss, angeordnet sein.

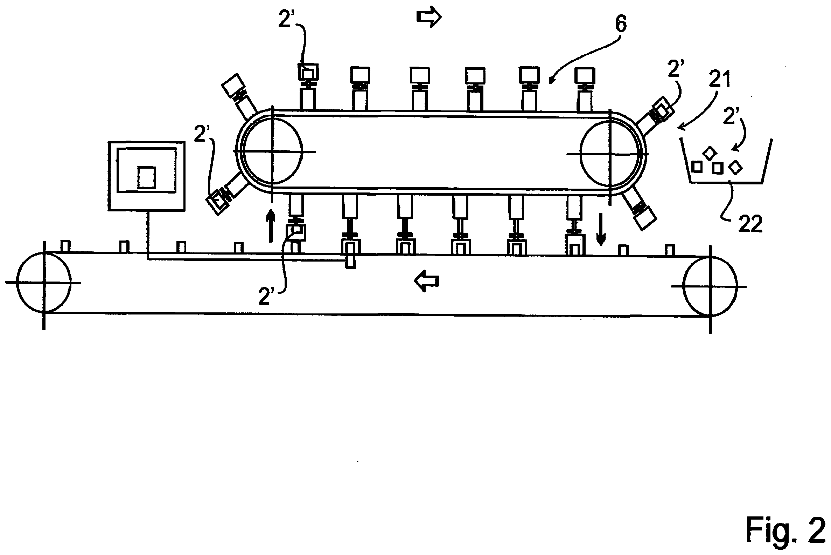

Die Ausschleusung kann, vgl. Fig. 2, durch Mitnehmen einer undichten Verpackung 2' am Endlosförderer 6 bis zu einer Abwurfstelle 21 in einen Behälter 22 oder dergleichen erfolgen. Hierzu können am Endlosförderer 6 an entsprechenden Stellen, beispielhaft dargestellt in den Dichtkörpern 5, Saugglocken oder Greifer oder dergleichen vorgesehen sein, die undichte Verpackungen 2' aufnehmen und abgeben können.

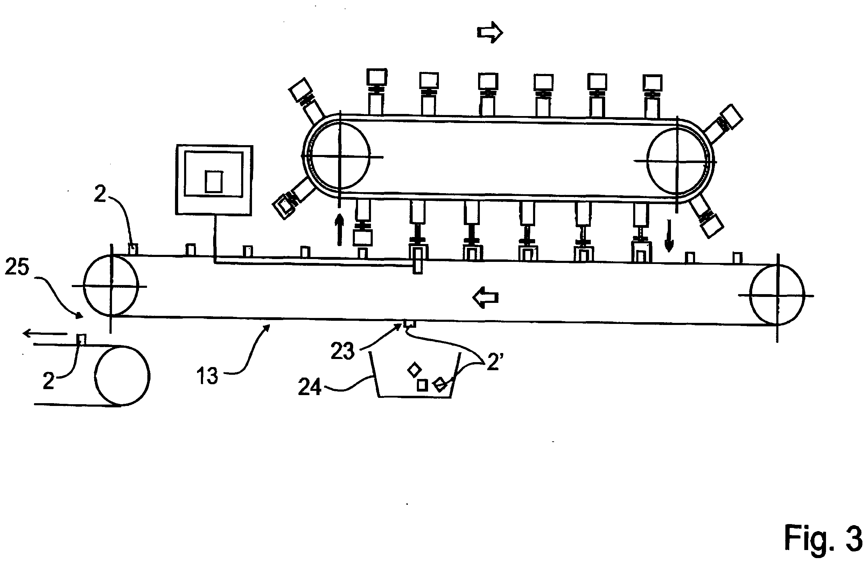

Bei der in Fig. 3 dargestellten Ausgestaltung werden undichte Verpackungen 2' am Endlosförderer 13 bis zu einer Abwurfstelle 23 gehalten, wo sie in einen Behälter 24 fallen, während dichte Verpackungen an einer anderen Stelle 25 in weiterverarbeitet werden, beispielsweise einer Fördervorrichtung 26 zugeführt werden.

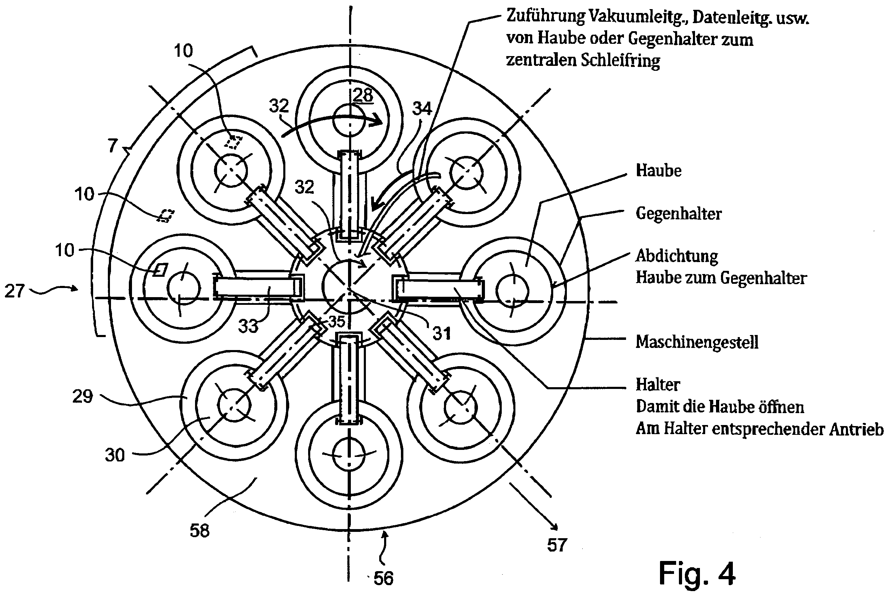

Bei der in Fig. 4 dargestellten Ausführungsform ist eine Rundprüfvorrichtung, hier in Form eines Rundtisches 27, vorgesehen, bei der mehrere Prüfkörper, hier in Form von Kammern 28, die aus auf Gegenhaltern 29 schließenden Hauben 30 gebildet werden, eine Achse 31 beispielsweise entlang der Pfeile 32 karusselartig umlaufen.

Jede der Kammern 28 kann über einen Mechanismus 33 (Hebel, pneumatischer Antrieb, elektrischer Antrieb usw.) insbesondere automatisch geöffnet und/oder geschlossen werden.

Besonders vorteilhaft ist, dass das Öffnen und Schließen der Kammern 28 beliebig ist, sofern für jede Kammer 28 ein separat steuer- oder betätigbarer Mechanismus 33 vorgesehen ist.

Jede Haube 30 wird im geschlossenen Zustand über eine Dichtung zum mitlaufenden Gegenhalter 29 abgedichtet und bildet so ein autarkes Volumen für den Messvorgang. Leitungen 34, von denen zur Vereinfachung nur eine dargestellt ist, für Energie, Vakuum, Druck, Daten und/oder einen Testimpuls sind radial nach innen geführt und von einem zentralen Verteiler 35 gespeist, z.B. einem Schleifring oder einer Vakuumverteilung oder dergleichen, wie sie aus WO 2008/104404 A2 oder WO 2010/089101 A1 bekannt sind, um Energie in eine Kette einzuspeisen.

Die Beschickung mit Gegenständen und auch die Entnahme kann beliebig angeordnet werden, z.B. an den Pfeilen 56, 57, da das Öffnen und Schließen der Kammern 28 durch einen separaten Antrieb gegeben ist. Dadurch ist diese Ausführungsform besonders flexibel. Zwischen Beschickung 56 und Entnahme 57 erstreckt sich die Prüfstrecke 7 und mindestens ein Sensor 10 kann am Maschinengestell 58 und/oder an und/oder in den Hauben 30 und/oder Gegenhaltern 29 angeordnet sein.

Besonders geeignet ist die Ausführungsform der Fig. 4 für eine vakuumbasierte Dichtheitsprüfung von Bechern undgroßvolumigen Verpackungseinheiten wie etwa Milchtüten. Sie wird bevorzugt waagerecht angeordnet.

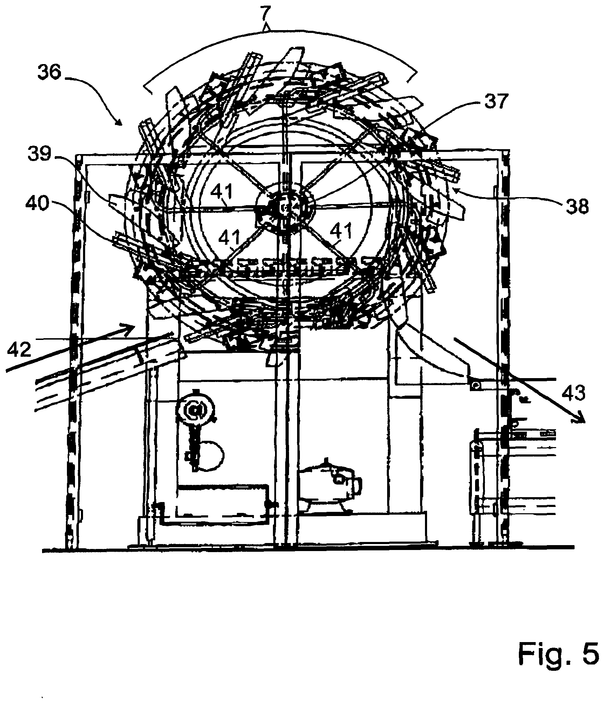

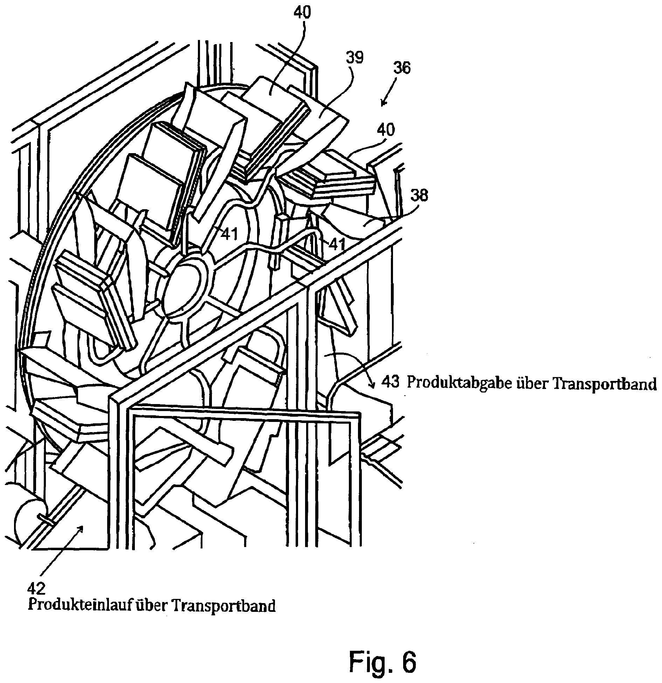

Die Ausführungsform gemäß Fig. 5 und 6 weist eine entfernt an ein Schaufelrad erinnernde Rundprüfvorrichtung 35 auf, die bevorzugt senkrecht oder gegenüber der Horizontalen geneigt, aber ggf. auch waagerecht, angeordnet ist. Ein Rad dreht sich um eine zentrale Achse 37 und weist am Außendurchmesser Kammern 38 auf, die Prüfkörper umfassen oder bilden. Während des Drehvorgangs fällt pro Einzelkammer eine Klappe 39 auf einen Unterteller 40 und dichtet diesen nach außen hin ab, ggf. unterstützt durch einen Betätigungsmechanismus. Die Abdichtung erfolgt vorzugsweise durch Dichtungen an der jeweiligen Zelle. Die Einzelkammern weisen Sensoren 10 auf und/oder werden an mindestens einem Sensor 10 entlang der Prüfstrecke 7 vorbeigeführt.

Leitungen 41 für Vakuum, Druck, elektrische oder sonstige Energie, elektrische oder sonstige Signale sind zur zentralen Achse 37 hin nach innen geführt und ähnlich wie bei der Ausgestaltung nach Fig. 3 über einen Schleifring oder eine Drehdurchführung oder dergleichen gespeist oder weitergeführt.

Im Gegensatz zur Ausführung nach Fig. 3 erfolgt die Beschickung und Entnahme an dafür vorgesehenen Punkten 42, 43 im System und nicht beliebig. Zur Entnahme kann die jeweilige Klappe 39 gravitationsbedingt und/oder durch Betätigung geöffnet werden, beispielsweise durch eine an geeigneter Stelle positionierten Finger, der die Klappe anhebt.

Die Ausführungsform gemäß Fig. 5 und 6 eignet sich besonders zur Dichtheitsprüfung von Chipstüten, Salzgebäck und dergleichen.

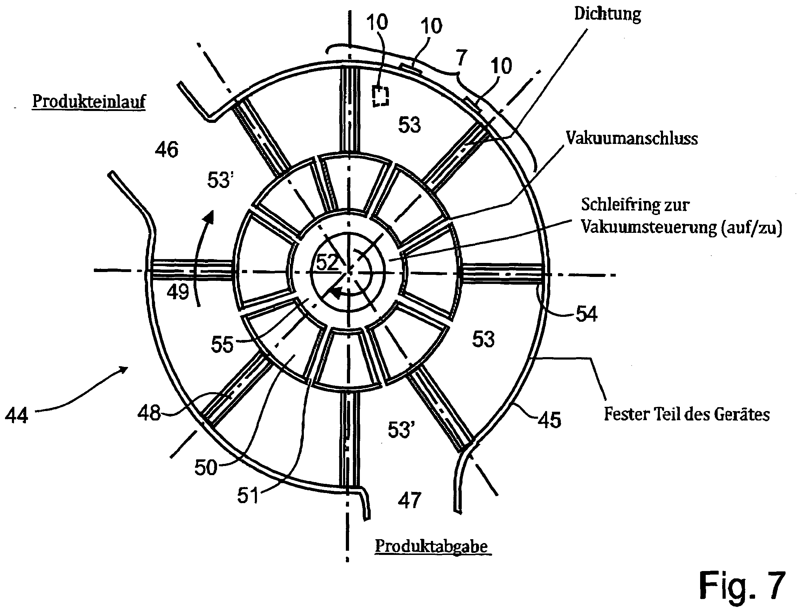

Die Vorrichtung 44 der Fig. 7 weist eine äußere Begrenzung 45 auf mit einer Zuführöffnung 46 sowie einer Abgabeöffnung 47 für kontinuierlich zugeführte bzw. abgegebene Gegenstände. Ein Rotor 50 mit Lamellen 48 läuft beispielsweise entlang des Pfeils 49 um. Der Rotor 50 weist Kanäle 51 auf, durch welche zentral über eine Vakuumeinspeisung 52 zugeführtes Vakuum in durch die Lamellen 48 gebildete Prüfkörper oder Kammern 53 gelangt. Die Lamellen 48 sind zweckmäßigerweise über eine Dichtung 54 gegenüber der Begrenzung 45 abgedichtet und im Inneren kann ein Schleifring 55 zur Steuerung der Vakuumabgabe vorgesehen sein; beispielsweise ist es nicht erforderlich, Vakuum in der jeweils zur Zuführöffnung 46 (oder Abgabeöffnung 47) offenen Kammer 53' zu erzeugen.

Ein oder mehrere Sensoren 10 können entlang der Prüfstrecke 7 angeordnet sein.

Diese Ausgestaltung ist besonders vorteilhaft verwendbar zur vakuumbasierten Dichtheitsprüfung von Schlauchbeutelverpackungen, z.B. Chipstüten, Salzgebäck usw.

## Patentansprüche

1. Verfahren zur vakuumbasierten Dichtheitsprüfung von in einem kontinuierlichen Strom (4) zugeführten Gegenständen, wobei entlang einer Prüfstrecke (7) ein Prüfkörper mit einem Gegenstand mitgeführt wird, **dadurch gekennzeichnet, dass** mittels eines Sensors (10) eine Reaktion auf eine Prüfung des Gegenstands erfasst wird, um festzustellen, ob der Gegenstand ein Qualitätskriterium erfüllt,wobei der Prüfkörper ein Dichtkörper (5) ist, wobei zur vakuumbasierten Dichtheitsprüfung der im kontinuierlichen Strom (4) zugeführten Gestände entlang der Prüfstrecke (7) der Dichtkörper (5) mit einem Gegenstand mitgeführt und mit Vakuum (9) beaufschlagt wird und mittels des Sensors (10) eine Reaktion auf die Beaufschlagung des Gegenstands mit Vakuum (9) erfasst wird, um festzustellen, ob der Gegenstand dicht oder undicht oder maßhaltig oder funktionsfähig ist.

2. Verfahren nach Anspruch 1, **dadurch gekennzeichnet, dass** der Prüfkörper den Sensor (10) umfasst; und/oder der Prüfkörper eine Prüfung des Gegenstands veranlasst; und/oder der Prüfkörper den Gegenstand mit einem Vakuum, einem Druck und/oder einem mechanischen, akustischen, optischen, thermischen und/oder elektrischen oder sonstigen Testimpuls beaufschlagt und/oder den Gegenstand oder einen Teil davon betätigt, auslenkt, befüllt, entleert, ein- und/oder ausschaltet, oder anderweitig stimuliert; und/oder
mittels des Sensors (10) eine optische, akustische, thermische, mechanische oder sonstige Reaktion des Gegenstands erfasst und auf ein Qualitätskriterium hin geprüft wird.

3. Verfahren nach einem der Ansprüche 1 bis 2, **dadurch gekennzeichnet, dass** als Reaktion auf eine Beaufschlagung mit Vakuum (9) eine Verformung der des Gegenstands, einer Verpackung (2) und/oder eines Spritzgußbauteils, ein Druckanstieg im Prüfkörper, eine verlangsamte Vakuumbildung im Prüfkörper, ein Gas im Prüfkörper, ein Geräusch im Prüfkörper und/oder eine optische, thermisch, akustisch und/oder mechanisch wahrnehmbare Reaktion oder das Ausbleiben einer derartigen Reaktion und/oder dergleichen erfasst wird.

4. Verfahren nach einem der Ansprüche 1 bis 3, **dadurch gekennzeichnet, dass**
der Gegenstand angehoben und/oder der Prüfkörper abgesenkt wird und/oder der Prüfkörper ein Prüfmittel betätigt; und/oder
die Gegenstände auf einer Unterglocke (14) angeordnet sind, der Dichtkörper (5) mit der Unterglocke (14) abdichtet und die Verpackung (2) zwischen Dichtkörper (5) und Unterglocke (14) eingeschlossen ist.

5. Verfahren nach einem der Ansprüche 1 bis 4, **dadurch gekennzeichnet, dass** der Strom auf einem Endlosförderer (13) zugeführt wird und/oder der Prüfkörper an einem Endlosförderer (6) umläuft.

6. Verfahren nach Anspruch 5, **dadurch gekennzeichnet, dass** der Endlosförderer (6; 13) einen integrierten Energiekanal aufweist, der mit pneumatischer Energie beaufschlagt wird, die als Vakuum zur Gasentleerung des als Dichtkörper (5) ausgestalteten Prüfkörpers entnommen wird; und/oder Gegenstände, die das Qualitätskriterium nicht erfüllen, wie etwa undichte Verpackungen (2') oder nicht maßhaltige Bauteile, auf dem Endlosförderer (6; 13) gehalten und im Rücklauf abgeworfen werden.

7. Verfahren nach einem der Ansprüche 1 bis 6, **dadurch gekennzeichnet, dass**
eine Rundprüfvorrichtung (27, 36, 44) verwendet wird, bei der mehrere Prüfkörper um eine Achse umlaufen, und/oder:
als Prüfkörper Kammern (28, 30, 53) oder Hauben verwendet werden, die ggf. über einen Mechanismus (33) geöffnet und/oder geschlossen werden können; und/oder
eine Leitung (34, 41) für Energie, Vakuum, Druck, Daten und/oder einen Testimpuls radial nach innen geführt und von einem zentralen Verteiler gespeist wird; und/oder
die Rundprüfvorrichtung waagerecht angeordnet wird; und/oder
die Rundprüfvorrichtung senkrecht oder gegenüber der Horizontalen geneigt angeordnet wird

8. Verfahren nach Anspruch 7, **dadurch gekennzeichnet, dass** der Prüfkörper als Dichtkörper ausgestaltet ist mit einer Klappe und einem Unterteller, wobei die Klappe nach Zuführen des Gegenstands unter Schwerkrafteinwirkung und/oder durch einen Aktuator betätigt auf den Unterteller fällt, und vor Abgabe des Gegenstands unter Schwerkrafteinwirkung und/oder durch einen Aktuator betätigt öffnet.

9. Verfahren nach einem der vorhergehenden Ansprüche, zur vakuumbasierten Bauteilprüfung von in einem kontinuierlichen Bauteilstrom (4) zugeführten Bauteilen (2), insbesondere Spritzgussteilen, **dadurch gekennzeichnet, dass** entlang einer Prüfstrecke (7) ein Dichtkörper (5) mit einem Bauteil (2) mitgeführt und mit Vakuum (9) beaufschlagt wird und mittels eines Sensors (10) eine Reaktion auf die Beaufschlagung des Bauteils (2) mit Vakuum (9) erfasst wird, um festzustellen, ob das Bauteil (2) eine Qualitätsanforderung erfüllt.

10. Verfahren nach einem der vorhergehenden Ansprüche, zur vakuumbasierten Dichtheitsprüfung von in einem kontinuierlichen Verpackungsstrom (4) zugeführten Verpackungen (2), insbesondere Lebensmittelverpackungen, **dadurch gekennzeichnet, dass** entlang einer Prüfstrecke (7) ein Dichtkörper (5) mit einer Verpackung (2) mitgeführt und mit Vakuum (9) beaufschlagt wird und mittels eines Sensors (10) eine Reaktion auf die Beaufschlagung der Verpackung (2) mit Vakuum (9) erfasst wird, um festzustellen, ob die Verpackung (2) dicht oder undicht ist.

11. Verfahren nach Anspruch 10, **dadurch gekennzeichnet, dass** als Reaktion auf die Beaufschlagung mit Vakuum (9) eine Verformung der Verpackung (2), ein Druckanstieg im Dichtkörper (5), eine verlangsamte Vakuumbildung im Dichtkörper (5), ein Gas im Dichtkörper (5) und/oder dergleichen erfasst wird; und/oder
die Verpackung (2) angehoben und/oder der Dichtkörper (5) abgesenkt wird; und/oder
die Verpackungen (2) auf einer Unterglocke (14) angeordnet sind, der Dichtkörper (5) mit der Unterglocke (14) abdichtet und die Verpackung (2) zwischen Dichtkörper (5) und Unterglocke (14) eingeschlossen ist; und/oder
der Verpackungsstrom (4) auf einem Endlosförderer (13) zugeführt wird und/oder der Dichtkörper (5) an einem Endlosförderer (6) umläuft; und/oder
der Endlosförderer (6; 13) einen integrierten Energiekanal aufweist, der mit pneumatischer Energie beaufschlagt wird, die als Vakuum zur Gasentleerung des Dichtkörpers (5) entnommen wird; und/oder
undichte Verpackungen (2') auf dem Endlosförderer (6; 13) gehalten und im Rücklauf abgeworfen werden.

12. Vorrichtung (1, 27, 36, 44) zur vakuumbasierten Dichtheitsprüfung von in einem kontinuierlichen Strom (4) zugeführten Gegenständen, wobei ein entlang einer Prüfstrecke (7) mit einem Gegenstand mitführbarer Prüfkörper vorgesehen ist, **dadurch gekennzeichnet, dass** ein Sensor (10) zum Erfassen einer Reaktion auf eine Prüfung des Gegenstands vorgesehen und mit einer Auswerteeinheit (11) gekoppelt ist, um festzustellen, ob der Gegenstand ein Qualitätskriterium erfüllt, wobei der Prüfkörper ein Dichtkörper (5) ist, wobei zur vakuumbasierten Dichtheitsprüfung der im kontinuierlichen Strom (4) zugeführten Gegenstände entlang der Prüfstrecke (7) der Dichtkörper (5) mit einem Gegenstand mitgeführt und mit Vakuum (9) beaufschlagt wird und mittels des Sensors (10) eine Reaktion auf die Beaufschlagung des Gegenstands mit Vakuum (9) erfasst wird, um festzustellen, ob der Gegenstand dicht oder undicht oder maßhaltig oder funktionsfähig ist.

13. Vorrichtung nach Anspruch 12, **dadurch gekennzeichnet, dass** der Prüfkörper den Sensor (10) umfasst; und/oder
der Prüfkörper zum Durchführen einer Prüfung des Gegenstands ausgestaltet ist; wobei der Prüfkörper den Gegenstand mit einem Vakuum, einem Druck und/oder einem mechanischen, akustischen, optischen, thermischen und/oder elektrischen oder sonstigen Testimpuls beaufschlagen und/oder den Gegenstand oder einen Teil davon betätigen, auslenken, befüllen, entleeren, ein- und/oder ausschalten, oder anderweitig stimulieren kann; und/oder
der Sensor (10) zum Erfassen einer optischen, akustischen, thermischen, mechanischen oder sonstigen Reaktion des Gegenstands ausgestaltet ist zur Prüfung auf ein Qualitätskriterium; und/oder
der Gegenstand eine Verpackung (2) ist und der Prüfkörper ein Dichtkörper (5) ist, wobei zur vakuumbasierten Dichtheitsprüfung von in einem kontinuierlichen Verpackungsstrom (4) zugeführten Verpackungen (2)entlang der Prüfstrecke (7) der Dichtkörper (5) mit einer Verpackung (2) mitgeführt und mit Vakuum (9) beaufschlagt wird und mittels des Sensors (10) eine Reaktion auf die Beaufschlagung der Verpackung (2) mit Vakuum (9) erfasst und mittels der Auswerteeinheit ausgewertet wird um festzustellen, ob die Verpackung (2) dicht oder undicht ist; und/oder der Sensor und/oder die Auswerteeinheit zum Erfassen, als Reaktion auf eine Beaufschlagung mit Vakuum (9), einer Verformung der des Gegenstands, insbesondere einer Verpackung (2) oder eines Spritzgußbauteils, eines Druckanstiegs im Prüfkörper, einer verlangsamten Vakuumbildung im Prüfkörper, eines Gases im Prüfkörper, eines Geräuschs im Prüfkörper und/oder einer optischen, thermischen, akustischen und/oder mechanisch wahrnehmbaren Reaktion oder eines Ausbleibens einer derartigen Reaktion und/oder dergleichen ausgestaltet ist; und/oder
Mittel zum Anheben des Gegenstands angehoben und/oder zum Absenken des Prüfkörpers vorgesehen sind und/oder der Prüfkörper ein Prüfmittel aufweist; und/oder
die Gegenstände auf einer Unterglocke (14) angeordnet sind, der Dichtkörper (5) mit der Unterglocke (14) abdichtet und die Verpackung (2) zwischen Dichtkörper (5) und Unterglocke (14) eingeschlossen ist; und/oder der Strom auf einem Endlosförderer (13) zugeführt wird und/oder der Prüfkörper an einem Endlosförderer (6) umläuft; und/oder
der Endlosförderer (6; 13) einen integrierten Energiekanal aufweist, der mit pneumatischer Energie beaufschlagt wird, die als Vakuum zur Gasentleerung des als Dichtkörper (5) ausgestalteten Prüfkörpers entnommen wird; und/oder
Gegenstände, die das Qualitätskriterium nicht erfüllen, wie etwa undichte Verpackungen (2') oder nicht maßhaltige Bauteile, auf dem Endlosförderer (6; 13) gehalten und im Rücklauf abgeworfen werden; und/oder
eine Rundprüfvorrichtung vorgesehen ist, bei der mehrere Prüfkörper um eine Achse umlaufen; wobei die Prüfkörper Kammern oder Hauben aufweisen, die über einen Mechanismus geöffnet und/oder geschlossen werden können; und/oder eine Leitung für Energie, Vakuum, Druck, Daten und/oder einen Testimpuls radial nach innen geführt und mit einem zentralen Verteiler gekoppelt ist; und/oder
die Rundprüfvorrichtung waagerecht oder im wesentlichen waagerecht angeordnet ist; und/oder
die Rundprüfvorrichtung senkrecht oder gegenüber der Horizontalen geneigt angeordnet ist, und/oder der Prüfkörper als Dichtkörper ausgestaltet ist mit einer Klappe und einem Unterteller, wobei die Klappe nach Zuführen des Gegenstands unter Schwerkrafteinwirkung und/oder durch einen Aktuator betätigt auf den Unterteller fällt, und vor Abgabe des Gegenstands unter Schwerkrafteinwirkung und/oder durch einen Aktuator betätigt öffnet.

14. Vorrichtung (1, 27, 36, 44)nach einem der vorhergehenden Ansprüche, zur kontinuierlichen vakuumbasierten Prüfung von in einem kontinuierlichen Strom (4) zugeführten Bauteilen, insbesondere Spritzgussteilen, **dadurch gekennzeichnet, dass** ein entlang einer Prüfstrecke (7) mit eine Bauteil mitführ- und mit Vakuum (9) beaufschlagbarer Dichtkörper (5) vorgesehen ist und ein Sensor (10) zum Erfassen einer Reaktion auf die Beaufschlagung der des Bauteils mit Vakuum (9) vorgesehen und mit einer Auswerteeinheit (11) gekoppelt ist, die anhand der erfassten Reaktion das Bauteil als maßhaltig oder nicht maßhaltig oder dergleichen erkennen kann; und/oder zur vakuumbasierten kontinuierlichen Dichtheitsprüfung von in einem kontinuierlichen Verpackungsstrom (4) zugeführten Verpackungen (2), wobei dass ein entlang einer Prüfstrecke (7) mit einer Verpackung (2) mitführ- und mit Vakuum (9) beaufschlagbarer Dichtkörper (5) vorgesehen ist und ein Sensor (10) zum Erfassen einer Reaktion auf die Beaufschlagung der Verpackung (2) mit Vakuum (9) vorgesehen und mit einer Auswerteeinheit (11) gekoppelt ist, die anhand der erfassten Reaktion die Verpackung (2) als dicht oder undicht erkennen kann; wobei:
der Sensor (10) als Reaktion auf die Beaufschlagung mit Vakuum (9) eine Verformung der Verpackung (2), ein Druckanstieg im Dichtkörper (5), eine verlagsamte Vakuumbildung im Dichtkörper (5), ein Gas im Dichtkörper (5) und/oder dergleichen erfasst kann; und/oder
ein Mechanismus zum Anheben der Verpackung und/oder ein Mechanismus (16) zum Absenken der Dichtkörper (5) vorgesehen ist; und/oder die Verpackungen (2) auf einer Unterglocke (14) angeordnet sind, der Dichtkörper (5) mit der Unterglocke (14) abdichtet und die Verpackung (2) zwischen Dichtkörper (5) und Unterglocke (14) eingeschlossen ist, und/oder
ein Endlosförderer (13) zum Zuführen des Verpackungsstroms (4) vorgesehen ist und/oder der Dichtkörper (5) an einem Endlosförderer (6) befestigt ist und mit diesem umläuft; und/oder
der Endlosförderer (6; 13) einen integrierten Energiekanal aufweist, der mit pneumatischer Energie beaufschlagt wird, die als Vakuum zur Gasentleerung des Dichtkörpers (5) entnommen wird; und/oder der Endlosförderer (6; 13) einen Haltemechanismus für die Verpackungen (2) aufweist und eine Steuerung vorgesehen ist, mit der undichte Verpackungen (2') auf dem Endlosförderer (6; 13) gehalten und im Rücklauf abgeworfen werden; und/oder
ein optischer Sensor, ein Drucksensor, ein Gassensor, ein Durchflusssensor, ein Druckschalter und/oder dergleichen zum Erfassen einer Reaktion auf die Beaufschlagung der Verpackung (2) mit Vakuum (9) vorgesehen sind.

## Claims

1. A method for the vacuum-based leak testing of objects fed in a continuous flow (4), wherein a test body with an object is carried along a test section (7), **characterised in that** a reaction to a test of the object is detected by means of a sensor (10), in order to establish whether the object meets a quality criterion, wherein the test body is a sealing body (5), wherein for the vacuum-based leak testing of the objects fed in the continuous flow (4) along with the test section (7), the sealing body (5) is carried along with an object and subjected to a vacuum (9) and a reaction of the object being acted upon by the vacuum (9) is detected by means of the sensor (10), in order to establish whether the object is tight or leaky or dimensionally stable or functioning.

2. The method according to claim 1, **characterised in that** the test body comprises the sensor (10);
and/or the test body initiates testing of the object; and/or the test body acts on the object with a vacuum, a pressure and/or a mechanical, acoustic, optical, thermal and/or electrical or other test pulse and/or actuates, deflects, fills, empties, switches on and/or switches off, or otherwise stimulates the object or a part thereof; and/or
detects an optical, acoustic, thermal, mechanical or other reaction of the object by means of the sensor (10) and a quality criterion is tested.

3. The method according to any one of claims 1 to 2, **characterised in that**, as a reaction to being acted upon by a vacuum (9), a deformation of the object, of a package (2) and/or of an injection moulded component, an increase in pressure in the test body, a decelerated vacuum formation in the test body, a gas in the test body, a noise in the test body and/or an optical, thermal, acoustic and/or mechanically perceptible reaction or the absence of such a reaction and/or suchlike is detected.

4. The method according to any one of claims 1 to 3,
**characterised in that**
the object is raised and/or the test body is lowered and/or the test body actuates a testing means; and/or the objects are arranged on a lower bell cover (14), the sealing body (5) is sealed with the lower bell cover (14) and the package (2) is enclosed between the sealing body (5) and the lower bell cover (14).

5. The method according to any one of claims 1 to 4, **characterised in that** the flow is fed on a continuous conveyor (13) and/or the test body circulates on a continuous conveyor (6).

6. The method according to claim 5, **characterised in that** the continuous conveyor (6; 13) comprises an integrated energy channel, which is acted upon by pneumatic energy, which is removed as a vacuum for emptying gas from the test body constituted as a sealing body (5); and/or the objects which do not meet the quality criterion, such as for example leaky packages (2') or components which are not dimensionally stable, are kept on the continuous conveyor (6; 13) and discarded in the return travel.

7. The method according to any one of claims 1 to 6,
**characterised in that**
a round testing device (27, 36, 44) is used, wherein a plurality of test bodies circulate about an axis, and/or:
chambers (28, 30, 53) or hoods are used as test bodies, which can be opened and/or closed in each case by a mechanism (33);
and/or
a line (34, 41) for energy, vacuum, pressure, data and/or a test pulse is run radially inwards and is fed by a central distributor; and/or
the round testing device is arranged horizontally; and/or
the round testing device is arranged vertically or inclined with respect to the horizontal.

8. The method according to claim 7, **characterised in that** the test body is constituted as a sealing body with a flap and a saucer, wherein the flap falls onto the saucer after the feeding of the object under the effect of gravity and/or actuated by an actuator, and opens before release of the object under the effect of gravity and/or actuated by an actuator.

9. The method according to any one of the preceding claims, for the vacuum-based component testing of components (2), in particular injection moulded parts, fed in a continuous component flow (4), **characterised in that** a sealing body (5) with a component (2) is carried along a test section (7) and acted upon by a vacuum (9) and a reaction of the component (2) to being acted upon by a vacuum (9) is detected by means of a sensor (10), in order to establish whether the component (2) meets a quality requirement.

10. The method according to any one of the preceding claims, for the vacuum-based leak testing of packages (2), in particular foodstuff packages, fed in a continuous package flow (4), **characterised in that** a sealing body (5) with a package (2) is carried along a test section (7) and acted upon by a vacuum (9) and a reaction of the package (2) being acted upon by a vacuum (9) is detected by means of a sensor (10), in order to establish whether the package (2) is tight or leaky.

11. The method according to claim 10, **characterised in that**, as a reaction to being acted upon by a vacuum (9), a deformation of the package (2), an increase in pressure in the sealing body (5), a decelerated vacuum formation in the sealing body (5), a gas in the sealing body (5) and/or suchlike is detected; and/or the package (2) is raised and/or the sealing body (5) is lowered; and/or
the packages (2) are arranged on a lower bell cover (14), the sealing body (5) is sealed with the lower bell cover (14) and the package (2) is enclosed between the sealing body (5) and the lower bell cover (14); and/or
the package flow (4) is fed on a continuous conveyor (13) and/or the sealing body (5) circulates on a continuous conveyor (6); and/or
the continuous conveyor (6; 13) comprises an integrated energy channel, which is acted upon by pneumatic energy, which is removed as a vacuum for emptying gas from the sealing body (5); and/or
leaky packages (2') are kept on the continuous conveyor (6; 13) and discarded in the return travel.

12. A device (1, 27, 36, 44) for the vacuum-based leak testing of objects fed in a continuous flow (4), wherein a test body which can be carried with the object along a test section (7) is provided, **characterised in that** a sensor (10) is provided for detecting a reaction to a test on the object and is coupled with an evaluation unit (11), in order to establish whether the object meets a quality criterion, wherein the test body is a sealing body (5), wherein for the vacuum-based leak testing of the objects fed in the continuous flow (4) along the test section (7), the sealing body (5) is carried along with an object and subjected to a vacuum (9) and a reaction of the object being acted upon by the vacuum (9) is detected by means of the sensor (10), in order to establish whether the object is tight or leaky or dimensionally stable or functioning.

13. The device according to claim 12, **characterised in that** the test body comprises the sensor (10); and/or the test body is designed to carry out the testing of object, wherein the test body can act on the object with a vacuum, a pressure, and/or a mechanical, acoustic, optical, thermal and/or electrical or other test pulse and/or can actuate, deflect, fill, empty, switch on and/or switch off, or otherwise stimulate the object or a part thereof; and/or
the sensor (10) for detecting an optical, acoustic, thermal, mechanical or other reaction of the object is constituted for testing for a quality criterion; and/or
the object is a package (2) and the test body is a sealing body (5), wherein for the vacuum-based leak testing of packages (2) fed on a continuous package flow (4), the sealing body (5) with a package (2) is carried along the test section (7) and acted upon by a vacuum (9) and a reaction of the package (2) to being acted upon by a vacuum (9) is detected by means of a sensor (10) and is evaluated by means of the evaluation unit, in order to establish whether the package (2) is tight or leaky; and/or
the sensor and/or the evaluation unit is designed for detecting, as a reaction to being acted upon by a vacuum (9), a deformation of the object, of a package (2) and/or of an injection moulded component, an increase in pressure in the test body, a decelerated vacuum formation in the test body, a gas in the test body, a noise in the test body and/or an optical, thermal, acoustic and/or mechanically perceptible reaction or the absence of such a reaction and/or suchlike; and/or
means are provided for raising the object and/or for lowering the test body and/or the test body comprises a testing means; and/or
the objects are arranged on a lower bell cover (14), the sealing body (5) is sealed with the lower bell cover (14) and the package (2) is enclosed between the sealing body (5) and the lower bell cover (14); and/or the flow is fed on a continuous conveyor (13) and/or the test body circulates on a continuous conveyor (6); and/or
the continuous conveyor (6; 13) comprises an integrated energy channel, which is acted upon by pneumatic energy, which is removed as a vacuum for emptying gas from the test body constituted as a sealing body (5); and/or
objects which do not meet the quality criterion, such as for example leaky packages (2') or components which are not dimensionally stable, are kept on the continuous conveyor (6; 13) and discarded in the return travel; and/or
a round testing device is provided, wherein a plurality of test bodies comprise chambers or hoods, which can be opened and/or closed by means of a mechanism; and/or
a line for energy, vacuum, pressure, data and/or a test pulse is run radially inwards and is coupled with a central distributor; and/or
the round testing device is arranged horizontally or essentially horizontally; and/or
the round testing device is arranged vertically or inclined with respect to the horizontal, and/or the test body is constituted as a sealing body with a flap and a saucer, wherein the flap falls onto the saucer after the feeding of the object under the effect of gravity and/or actuated by an actuator, and opens before release of the object under the effect of gravity and/or actuated by an actuator.

14. The device (1, 27, 36, 44) according to any one of the preceding claims, for the continuous vacuum-based testing of components, in particular injection moulded parts, fed in a continuous flow (4), **characterised in that** a sealing body (5), which can be carried with a component along a test section (7) and can be acted upon by a vacuum (9), is provided and a sensor (10) is provided for detecting a reaction of the component acted upon by a vacuum (9) and is coupled with an evaluation unit (11), which with the aid of the detected reaction can recognise the component as dimensionally stable or not dimensionally stable or suchlike; and/or for the vacuum-based continuous leak testing of packages (2) fed in a continuous package flow (4), wherein a sealing body (5) which can be carried with a package (2) along a test section (7) and can be acted upon by a vacuum (9) is provided and a sensor (10) is provided for detecting a reaction of the package (2) acted upon by a vacuum (9) and is coupled with an evaluation unit (11), which with the aid of the detected reaction can recognise the package (2) as tight or leaky; wherein:
the sensor (10) can detect, as a reaction to being acted upon by a vacuum (9), a deformation of a package (2), an increase in pressure in the sealing body (5), a decelerated vacuum formation in the sealing body (5), a gas in the sealing body (5) and/or suchlike; and/or
a mechanism for raising the package and/or a mechanism (16) for lowering the sealing body (5) is provided; and/or
the packages (2) are arranged on a lower bell cover (14), the sealing body (5) is sealed with the lower bell cover (14) and the package (2) is enclosed between the sealing body (5) and the lower bell cover (14); and/or
a continuous conveyor (13) for feeding the package flow (4) is provided and/or the sealing body (5) is fixed on a continuous conveyor (6) and circulates with the latter; and/or
the continuous conveyor (6; 13) comprises an integrated energy channel, which is acted upon by pneumatic energy, which is removed as a vacuum for emptying gas from the sealing body (5); and/or
the continuous conveyor (6; 13) comprises a holding mechanism for the packages (2) and a control is provided with which leaky packages (2') are kept on the continuous conveyor (6; 13) and discarded in the return travel; and/or
an optical sensor, a pressure sensor, a gas sensor, a flow sensor, a pressure switch and/or suchlike for detecting a reaction of the package (2) to being acted upon by a vacuum (9).

## Revendications

1. Procédé de contrôle d'étanchéité basé sur du vide d'objets acheminés en un flux continu (4), un corps de contrôle étant acheminé conjointement avec un objet le long d'un parcours de contrôle (7), **caractérisé en ce que**, au moyen d'un capteur (10), une réaction à un contrôle de l'objet est détectée pour constater si l'objet répond à un critère de qualité, le corps de contrôle étant un corps d'étanchéité (5), dans lequel, pour le contrôle d'étanchéité des objets acheminés en flux continu (4) le long du parcours de contrôle (7), le corps d'étanchéité (5) est acheminé conjointement avec un objet et sollicité avec du vide (9) et que, au moyen du capteur (10), une réaction à la sollicitation de l'objet avec du vide (9) est détectée pour constater si l'objet est étanche ou non étanche ou stable dimensionnellement ou apte à fonctionner.

2. Procédé selon la revendication 1, **caractérisé en ce que**
le corps de contrôle comprend le capteur (10) ; et/ou le corps de contrôle initie un contrôle de l'objet ; et/ou
le corps de contrôle sollicite l'objet avec un vide, une pression et/ou une impulsion de test mécanique, acoustique, optique, thermique et/ou électrique ou autre et/ou actionne, dévie, remplit, vide, active et/ou ou désactive ou stimule autrement l'objet ou une partie de celui-ci ; et/ou,
au moyen du capteur (10), une réaction optique, acoustique, thermique, mécanique ou autre de l'objet est détectée et fait l'objet d'un contrôle d'un critère de qualité.

3. Procédé selon une des revendications 1 à 2, **caractérisé en ce que**, en tant que réaction à une sollicitation avec du vide (9), une déformation de l'objet, d'un emballage (2) et/ou d'une pièce moulée par injection, une hausse de pression dans le corps de contrôle, une formation ralentie de vide dans le corps de contrôle, un gaz dans le corps de contrôle, un bruit dans le corps de contrôle et/ou une réaction perceptible de manière optique, thermique, acoustique et/ou mécanique ou l'absence d'une telle réaction et/ou similaire est détecté.

4. Procédé selon une des revendications 1 à 3, **caractérisé en ce que**
l'objet est soulevé et/ou le corps de contrôle est abaissé et/ou le corps de contrôle actionne un moyen de contrôle ; et/ou
les objets sont disposés sur une sous-cloche (14), le corps d'étanchéité (5) établit une étanchéité avec la sous-cloche (14) et l'emballage (2) est intégré entre le corps d'étanchéité (5) et la sous-cloche (14).

5. Procédé selon une des revendications 1 à 4, **caractérisé en ce que** le flux est acheminé sur un convoyeur continu (13) et/ou le corps de contrôle tourne au niveau d'un convoyeur continu (6).

6. Procédé selon la revendication 5, **caractérisé en ce que** le convoyeur continu (6 ; 13) présente un canal énergétique intégré qui est sollicité avec de l'énergie pneumatique qui est prélevée sous forme de vide pour purger le gaz du corps de contrôle réalisé sous forme d'un corps d'étanchéité (5) ; et/ou que les objets qui ne remplissent pas le critère de qualité, comme les emballages non étanches (2') ou les pièces non stables dimensionnellement, sont maintenus sur le convoyeur continu (6 ; 13) et jetés dans le reflux.

7. Procédé selon une des revendications 1 à 6, **caractérisé en ce**
**qu'**on utilise un dispositif de contrôle rond (27,36,44) dans lequel plusieurs corps de contrôle tournent autour d'un axe, et/ou :
**qu'**on utilise comme corps de contrôle des chambres (28,30,53) ou des capots qui peuvent le cas échéant être ouverts et/ou fermés par un mécanisme (33) ; et/ou
**qu'**une conduite (34, 41) pour de l'énergie, du vide, de la pression, des données et/ou une impulsion de test est guidée radialement vers l'intérieur et stockée dans un distributeur central ; et/ou
**que** le dispositif de contrôle rond est disposé horizontalement ; et/ou
**que** le dispositif de contrôle rond est disposé verticalement ou incliné par rapport à l'horizontale.

8. Procédé selon la revendication 7, **caractérisé en ce que** le corps de contrôle se présente sous forme d'un corps d'étanchéité doté d'un volet et d'un plateau inférieur, le volet, après acheminement de l'objet sous exercice de la force de gravité et/ou une fois actionné par un actionneur, tombant sur le plateau inférieur et, avant le dépôt de l'objet, s'ouvrant sous l'effet de la force de gravité et/ou une fois actionné par un actionneur.

9. Procédé selon une des revendications précédentes, destiné au contrôle basé sur le vide de pièces (2) acheminées en un flux continu de pièces (4), en particulier de pièces moulées par injection, **caractérisé en ce que**, le long d'un parcours de contrôle (7), un corps d'étanchéité (5) est acheminé conjointement avec une pièce (2) et sollicité avec du vide (9) et que, au moyen d'un capteur (10), une réaction à la sollicitation de la pièce (2) avec du vide (9) est 32 pour constater si la pièce (2) répond à une exigence de qualité.

10. Procédé selon une des revendications précédentes, destiné au contrôle basé sur le vide d'emballages (2) acheminés en un flux continu d'emballages (4), en particulier d'emballages alimentaires, **caractérisé en ce que**, le long d'un parcours de contrôle (7), un corps d'étanchéité (5) est acheminé conjointement avec un emballage (2) et sollicité avec du vide (9) et que, au moyen d'un capteur (10), une réaction à la sollicitation de l'emballage (2) avec du vide (9) est détectée pour constater si l'emballage (2) est étanche ou non étanche.

11. Procédé selon la revendication 10, **caractérisé en ce que**, en tant que réaction à la sollicitation avec du vide (9), une déformation de l'emballage (2), une hausse de pression dans le corps d'étanchéité (5), une formation ralentie de vide dans le corps d'étanchéité (5), un gaz dans le corps d'étanchéité (5) et/ou similaire est détecté ; et/ou
l'emballage (2) est soulevé et/ou le corps d'étanchéité (5) est abaissé ; et/ou
les emballages (2) sont disposés sur une sous-cloche (14), le corps d'étanchéité (5) établit une étanchéité avec la sous-cloche (14) et l'emballage (2) est intégré entre le corps d'étanchéité (5) et la sous-cloche (14), et/ou
le flux d'emballages (4) est acheminé au niveau d'un convoyeur continu (13) et/ou le corps d'étanchéité (5) tourne au niveau d'un convoyeur continu (6) ; et/ou le convoyeur continu (6 ; 13) présente un canal énergétique intégré qui est sollicité avec de l'énergie pneumatique qui est prélevée sous forme de vide pour purger le gaz du corps d'étanchéité (5) ; et/ou
les emballages non étanches (2') sont maintenus sur le convoyeur continu (6 ; 13) et jetés dans le reflux.

12. Dispositif (1,27,36,44) de contrôle d'étanchéité basé sur du vide d'objets acheminés en un flux continu (4), un corps de contrôle pouvant être acheminé avec un objet le long d'un parcours de contrôle (7) étant prévu, **caractérisé en ce qu'**un capteur (10) est prévu pour détecter une réaction à un contrôle de l'objet et couplé à une unité d'exploitation (11) pour constater si l'objet répond à un critère de qualité, le corps de contrôle étant un corps d'étanchéité (5), dans lequel, pour le contrôle d'étanchéité des objets acheminés en flux continu (4) le long du parcours de contrôle (7), le corps d'étanchéité (5) est acheminé conjointement avec un objet et sollicité avec du vide (9) et, au moyen du capteur (10), une réaction à la sollicitation de l'objet avec du vide (9) est détectée pour constater si l'objet est étanche ou non étanche ou stable dimensionnellement ou apte à fonctionner.

13. Dispositif selon la revendication 12, **caractérisé en ce que** le corps de contrôle comprend le capteur (10) ; et/ou
que le corps de contrôle est conçu pour réaliser un contrôle de l'objet ; le corps de contrôle pouvant solliciter l'objet avec un vide, une pression et/ou une impulsion de test mécanique, acoustique, optique, thermique et/ou électrique ou autre et/ou actionner, dévier, remplir, vider, activer et/ou ou désactiver ou stimuler autrement l'objet ou une partie de celui-ci ; et/ou,
que le capteur (10) est conçu pour détecter une réaction optique, acoustique, thermique, mécanique ou autre de l'objet pour faire un contrôle d'un critère de qualité ; et/ou
que l'objet est un emballage (2) et que le corps de contrôle est un corps d'étanchéité (5), dans lequel, pour le contrôle d'étanchéité basée sur du vide d'emballages (2) acheminés en un flux continu d'emballages (4) le long du parcours de contrôle (7), le corps d'étanchéité (5) est acheminé conjointement avec un emballage (2) et sollicité avec du vide (9) et que, au moyen du capteur (10), une réaction à la sollicitation de l'emballage (2) avec du vide (9) est détectée et exploitée au moyen de l'unité d'exploitation pour constater si l'emballage (2) est étanche ou non étanche ; et/ou
que le capteur et/ou l'unité d'exploitation sont conçus pour détecter, en tant que réaction à une sollicitation avec du vide (9), une déformation de l'objet, en particulier d'un emballage (2) ou d'une pièce moulée par injection, une hausse de pression dans le corps de contrôle, une formation ralentie de vide dans le corps de contrôle, un gaz dans le corps de contrôle, un bruit dans le corps de contrôle et/ou une réaction perceptible de manière optique, thermique, acoustique et/ou mécanique ou une absence d'une telle réaction et/ou similaire ; et/ou que des moyens de soulèvement de l'objet et/ou d'abaissement du corps de contrôle sont prévus et/ou le corps de contrôle présente un moyen de contrôle ; et/ou
que les objets sont disposés sur une sous-cloche (14), le corps d'étanchéité (5) établit une étanchéité avec la sous-cloche (14) et l'emballage (2) est intégré entre le corps d'étanchéité (5) et la sous-cloche (14) ; et/ou
que le flux est acheminé sur un convoyeur continu (13) et/ou que le corps de contrôle tourne au niveau d'un convoyeur continu (6) ; et/ou
que le convoyeur continu (6 ; 13) présente un canal énergétique intégré qui est sollicité avec de l'énergie pneumatique qui est prélevée sous forme de vide pour la purge du gaz du corps de contrôle réalisé sous forme de corps d'étanchéité (5) ; et/ou que les objets qui ne remplissent pas le critère de qualité, comme des emballages non étanches (2') ou des pièces non stables dimensionnellement, sont maintenus sur le convoyeur continu (6 ; 13) et jetés dans le reflux ; et/ou
qu'il est prévu un dispositif de contrôle rond dans lequel plusieurs corps de contrôle tournent autour d'un axe, les corps de contrôle présentant des chambres ou des capots qui peuvent être ouverts et/ou fermés par un mécanisme ; et/ou
qu'une conduite pour de l'énergie, du vide, de la pression, des données et/ou une impulsion de test est guidée radialement vers l'intérieur et couplée à un répartiteur central ; et/ou
que le dispositif de contrôle rond est disposé horizontalement ou sensiblement verticalement ; et/ou que le dispositif de contrôle rond est disposé verticalement ou incliné par rapport à l'horizontale, et/ou que le corps de contrôle rond est réalisé sous forme d'un corps d'étanchéité comportant un volet et un plateau inférieur, le volet, après acheminement de l'objet sous exercice de la force de gravité et/ou une fois actionné par un actionneur, tombant sur le plateau inférieur et, avant le dépôt de l'objet, s'ouvrant sous l'effet de la force de gravité et/ou une fois actionné par un actionneur.

14. Dispositif (1,27,36,44) selon une des revendications précédentes destiné au contrôle d'étanchéité continu basé sur du vide de pièces acheminées en un flux continu (4), en particulier de pièces moulées par injection, **caractérisé en ce qu'**un corps d'étanchéité (5) pouvant être acheminé avec une pièce et sollicité avec du vide (9) le long d'un parcours de contrôle (7) est prévu et qu'un capteur (10) est prévu pour détecter une réaction à la sollicitation de la pièce avec du vide (9) et couplé à une unité d'exploitation (11) qui, sur la base de la réaction détectée, peut reconnaître la pièce comme stable dimensionnellement ou non stable dimensionnellement ; et/ou pour le contrôle d'étanchéité continu basé sur du vide d'emballages (2) acheminés en un flux continu (4) d'emballages, un corps d'étanchéité (5) pouvant être acheminé le long d'un parcours de contrôle (7) avec un emballage (2) et sollicité avec du vide (9) étant prévu et un capteur (10) étant prévu pour détecter une réaction à la sollicitation de l'emballage (2) avec du vide (9) et couplé à une unité d'exploitation (11) qui, sur la base de la réaction détectée, peut reconnaître l'emballage (2) comme étanche ou non étanche ;
le capteur (10), en réaction à la sollicitation avec du vide (9), pouvant détecter une déformation de l'emballage (2), une hausse de pression dans le corps d'étanchéité (5), une formation ralentie de vide dans le corps d'étanchéité (5), un gaz dans le corps d'étanchéité (5) et/ou similaire ; et/ou qu'un mécanisme de soulèvement de l'emballage et/ou un mécanisme (16) d'abaissement des corps d'étanchéité (5) est prévu ; et/ou
que les emballages (2) sont disposés sur une sous-cloche (14), que le corps d'étanchéité (5) établit l'étanchéité avec la sous-cloche (14) et que l'emballage (2) est intégré entre le corps d'étanchéité (5) et la sous-cloche (14), et/ou qu'un convoyeur continu (13) est prévu pour acheminer le flux d'emballages (4) et/ou que le corps d'étanchéité (5) est fixé à un convoyeur continu (6) et tourne avec celui-ci ; et/ou
que le convoyeur continu (6 ; 13) présente un canal énergétique intégré qui est sollicité avec de l'énergie pneumatique qui est prélevée sous forme de vide pour la purge du gaz du corps d'étanchéité (5) ; et/ou
que le convoyeur continu (6 ; 13) présente un mécanisme d'arrêt pour les emballages (2) et qu'il est prévu une commande grâce à laquelle les emballages non étanches (2') sont maintenus sur le convoyeur continu (6 ; 13) et jetés dans le reflux ; et/ou qu'un capteur optique, un capteur de pression, un capteur de gaz, un capteur de débit, un interrupteur de pression et/ou similaires sont prévus pour enregistrer une réaction à la sollicitation de l'emballage (2) avec du vide (9).
